# EUROPEAN PATENT SPECIFICATION

(11) **EP 2 327 955 B1**
(45) Date of publication and mention of the grant of the patent: **16.03.2016**
(21) Application number: 09811139.6
(22) Date of filing: 29.07.2009
(51) Int. Cl.: G01N 21/45, G01N 33/543, G01N 21/05

(54) **Optical detection system for labelling-free high-sensitivity bioassays**
Optisches Erkennungssystem für markierungsfreie Bioassays von hoher Empfindlichkeit
Système de détection optique pour essais biologiques à haute sensibilité sans marquage

(30) Priority: 05.09.2008 ES 200802565
(43) Date of publication of application: 01.06.2011
(73) Proprietor: Universidad Politécnica De Madrid, 28040 Madrid (ES)
(72) Inventor: Holgado Bolaños, Miguel, E-28040 Madrid (ES); Casquel Del Campo, Rafael, E-28040 Madrid (ES); Molpeceres Álvarez, Carlos, E-28008 Madrid (ES); Ocaña Moreno, José Luis, E-28040 Madrid (ES); Lagunas Heras, María Fé, E-28040 Madrid (ES); Morales Furio, Miguel, E-28040 Madrid (ES)
(74) Representative: ABG Patentes, S.L.
(86) International application number: PCT/ES2009/000405
(87) International publication number: WO 2010/026269

(56) References cited:
- EP-A2- 1 892 518
- WO-A1-2008/101348
- US-A1- 2003 112 443
- US-A1- 2008 119 701
- US-A1- 2008 129 981
- US-A1- 2008 219 615
- US-A1- 2008 252 890
- US-A1- 2008 304 073
- BERGSTEIN D A ET AL: "Resonant Cavity Imaging: A Means Toward High-Throughput Label-Free Protein Detection", IEEE JOURNAL OF SELECTED TOPICS IN QUANTUM ELECTRONICS, IEEE SERVICE CENTER, PISCATAWAY, NJ, US, vol. 14, no. 1, 1 January 2008 (2008-01-01), pages 131-139, XP011204166, ISSN: 1077-260X
- BERGSTEIN D A ET AL: "Resonant cavity imaging biosensor", LIGHTWAVE TECHNOLOGIES IN INSTRUMENTATION AND MEASUREMENT CONFERENCE, 2004. PROCEEDINGS OF THE PALISADES, NY, USA OCTOBER 19-20, 2004, PISCATAWAY, NJ, USA,IEEE, 19 October 2004 (2004-10-19), pages 101-105, XP010752123, DOI: 10.1109/LTIMC.2004.1371002 ISBN: 978-0-7803-8723-2
- BERGSTEIN D A ET AL: "Silicon substrtates with buried distributed bragg reflectors for biosensing", SEMICONDUCTOR DEVICE RESEARCH SYMPOSIUM, 2005 INTERNATIONAL BETHESDA, MARYLAND, USA DEC. 7-9, 2005, PISCATAWAY, NJ, USA,IEEE, 7 December 2005 (2005-12-07), pages 262-263, XP010895160, DOI: 10.1109/ISDRS.2005.1596085 ISBN: 978-1-4244-0083-6

## Description

The object of the invention is based on the design and holistic development of the bio-detection system by combining the interferometric and resonant advantages of novel photonic structures with optical interrogation techniques such as spectrometry, ellipsometry as well as simultaneous angle resolved reflectance and/or transmittance in micro/sub-micro domains, making the system as a whole offer maximum sensitivity and robustness, eliminating ambiguities in the optical interrogation process. Said cavities are compatible with and adapted to the necessary fluid requirements to carry out labelling-free bioassays. Besides, the measurement result will be obtained in a short time interval, in a few seconds. The field of application of the present invention relates to the field of engineering and physics, and more specifically, to the biophotonics area, materials, optics and micro-nano technology.

### BACKGROUND OF THE INVENTION

Sectors such as the biomedical and clinical sectors are continuously demanding better and more efficient analysis techniques and tools for identifying diseases, developing new medicine and improving diagnosis efficiency. In order to achieve this object, the detection systems comprising optical materials and photonic components, as a base for the development of optical biodetection systems, are positioning themselves as a significant alternative with great potential (Sensitive optical biosensors for unlabeled targets: A review, Anal. Chim. Record 620, pp. 8-26, 2008. doi:10.1016/j.aca.2008.05.022*.*). Apart from the high sensitive degree that these systems can achieve, they offer other advantages such as the fact that: they are noninvasive, do not pose any risk of electric shock or explosion and are immune to electromagnetic interference. A key aspect for the development of these systems is the degree of development of the miniaturization technology at a micro and nano scale, capable of integrating mechanical, fluid and photonic components for developing efficient detection biosystems.

The process of discovering whether particular pathogens or proteins are present in a particular analyte in the biodetection optical systems is performed by immobilizing the bioreceptors on specific areas of the sensor. Once the immobilization process has been performed, the test analytes are introduced into said areas, which will react if they have particular contents of pathogens or proteins that are captured by the above mentioned immobilized molecular receptors, in a process that is called molecular recognition (MR). Most of the time this recognition process is monitored by techniques requiring labelling elements such as fluorescent dyes, radioactive isotopes or magnetic particles, among others, for its transduction. However, the chemistry associated with the labelling process involves more time and higher costs. By contrast, the refractometric optical biosensors do not require this type of labelling for the detection, which is called label-free biosensing systems. Label-free biosensing systems are expected to become more important in areas such as the clinical, bio-pharmaceutical, environmental, and security sectors among others due to their sensitivity and capacity for performing real-time analysis, saving reactives and analytes and, ultimately, lowering the cost per analysis.

The technology of miniaturization and integration at micro-nano scale is attaining promising results for the development of new sensors for label-free biodetection. There have been developed nanomechanical devices such as, for example, resonant microcantilevers, and acoustic resonators based on MEMS (Micro Electro Mechanical Systems). However, the sensitivity they offer is considerably lower than integrated photonic devices. The integrated photonic devices for their application as refractometric biosensors are especially relevant when they are manufactured using conventional techniques of micro-nanomanufacturing planar technology, which allows for miniaturization, cost reduction, large-scale production, mechanical stability and the possibility of manufacturing optical, fluid, electrical and photonic components necessary for achieving high sensitivity monolithic and compact devices, and with capacity for simultaneously measuring different analytes. Refractometric optical detection systems use as transduction the effect that generates both the immobilization of biomolecule and the molecular recognition in refractive index variations. Nowadays, there have been developed and tested refractometric optical biosensors made in micro/nanomanufacturing planar technology that usually use the evanescent field of a light mode guided by a high refractive index waveguide and are based on photonic structures such as the surface plasmon resonance (SPR), interferometers, resonators, diffraction networks and photonic crystals. However, this type of photonic devices usually needs complex coupling techniques, for example, between the waveguide and the fiber optic (for example, inverted narrowing guides and couplers based on diffraction networks) to obtain the information, which hinders the encapsulation and packaging thereof and drastically affects the device cost.

Some of these sensors, described below, have already been commercialized, others are on the commercialization phase, and some others have not been commercialized yet:

### 1.- Refractometric biosensors based on the Surface Plasmon Resonance (SPR):

Since its discovery in 1993, it has been one of the most successful optical techniques for label-free biosensors, being commercially accepted and distributed by, for example, the commercial firms Biacore (*www.biacore.com*) and Ibis Technologies (*www.Ibis-spr.nl,*) among others. The SPR technique is widely used for determining several types of biological interactions and hundreds of publications appear in the literature each year. There also exists a large number of patents, some of them are referred to as examples: US5492840, US2008193965, US2064090630, WO2008101348, WO2008054282, as well as scientific references *(*Analysis of the performance of interferometry, surface plasmon resonance and luminescence as biosensors and chemosensors, Review. Anal. Chim. Record 569 (1-2). pp. 1-20> 2006. doi.10.1016/j.aca.2006.03.058*.)* and *(*Surface plasmon resonance sensors: review, Sensors and Actuators B: Chemical 54 (1 -2), pp. 3-15, 1999. doi: 10.1016/S0925-4005(98)00321-9*.)*. The disadvantage of this system against the one that is proposed in the present invention is based on the fact that these systems are slower, cannot be miniaturized so as to achieve a high integration in the devices, they do not have high measuring cadence, and thus they have a higher cost per analysis.

### 2. Refractometric biosensors based on interferometers:

There exist several types of refractometric biosensors based on interferometry, among which it could be noted: Young interferometer, those based on diffraction networks and the Mach-Zehnder interferometers:
*2.1.- Young Interferometer (Y-I):* commercialized by Farfield Group (www.farfield-scientific.com), it offers high sensitivity. It consists of four dielectric layers, two of which are the sensitive area and the other two act as reference. The interferometry between the two signals allows determining the detection (US 7050176B1) and (A new quantitative optical biosensor for protein characterization, Biosensors and Bioelectronics 19 (4) pp. 383-390, 2003. doi:10.1016/S0956-5663(03)00203-3*)*. It can also use the polymerization for the excitation. However, it is a technique in which the possibility of integrating multiple chips is more complex compared to other techniques, being, therefore, the capacity of integration and the measuring cadence lower and the cost per analysis higher in comparison with the biodetection system that is proposed in the present invention.
*2.2 Based* on *diffraction networks*: For example, the waveguide grating couplers (WGC) based on diffraction networks: that have been used for commercializing several label-free biodetection systems. There are several companies that have commercialized this system (for example, Microvacum: www.microvacuum.com, among others). In these systems, waveguides are used as a basic element of their structures for the propagation of light and these systems are also based on the evanescent field for the detection. Within this group it can be noted the system of high cadence of measurement recently commercialized by Coming (http://www.coming.com/) based on diffraction network couplers. This system is capable of measuring every few seconds in standard wells with a capacity in the microlitres range per well. There follow some references to some existing patents relating to biosensors based on couplers and diffraction networks: US2008219892, EP1892518, GB2227089, as well as some scientific reference *(*Optical grating coupler biosensors, Biomaterials 23 (17), pp. 3699-3710, 2002. doi:10.1016/S0142-5 9612(02)00103-5*), (*Wavelength-Interrogated optical sensor for biomedical applications, Opt. Lett. 25 (7), pp. 463-465, 2000. doi:10.1364/OL.25.000463*)*. This system offers a high cadence solution but in comparison with the present invention, it is less robust, sensitive and the measuring wells have a larger volume than the ones that can be achieved with the present invention, being it possible to measure volumes in the femtolitre range.
2.3. - *Mach-Zehnder Interferometers (M-Z):* They potentially offer high sensitivity as label-free biosensors by having an internal reference for compensating refractive index fluctuations due to non-specific adsorption. Interferometric sensors provide a wide dynamic range compared to other integrated sensors. However, most of the research done still seems to be confined to laboratory level and as far as we know it has not been commercially exploited. These devices have the disadvantage of their expensive encapsulation and packaging for they require an fiber optic-waveguide coupling. The following can be consulted as scientific reference: J. Lightwave Technol. 16 (4), pp. 583-592, 1998*),* Sensors and actuators B: Chemical 92, pp.151-158, 2003. doi:10.1016/S0925-4005(03)00257-0 *and* Journal of Nanotechnology 14, pp. 907-912, 2003. doi: 10.108810957-4484/14/8/312*. Mach-Zehnder Interferometers (M-Z)* based on ARROW (Anti resonant reflecting optical waveguides) (GA) are a particular case: In this case the electrical field is confined into a waveguide and the evanescent field interacts with the biomolecules *(*Sensors and actuators B: Chemical 92, pp. 151-1 58, 2003. doi:10.1016/S0925-4005(03)00257-0*)*. ARROW hollow guides have been tested, with high sensitive. So far, as far as the authors of the present invention know, they have not been developed for commercial application.

### 3. Refractometric biosensors based on optical resonators:

The phenomenon of resonance is a concept that can be exploited for label-free bio-detection. In this type of devices, sensitivity is determined by the quality factor of the resonant cavities and their response to affinity reactions and molecular recognition that are generated therein. Among others, it is noted, as an emergent technique, the integrated devices based on micro-rings and micro-discs resonators (for example, US2004023396) in which the quality factor reported in the bibliography is very high. (for example, Integrated optics ring-resonator sensors for protein detection, Opt. Lett. 3 (24). pp. 3344-3346, 2005. doi: 10.1364/OL.30.003344). Other configurations may use resonant microspheres or another type of cavities, and it has been recently tested nano-slot waveguides where biomolecules are anchored inside them to improve their response (Label-free optical biosensing with slotwaveguides. Opt. Lett. 33, (7), pp:708-710, 2008. doi:10.1364/OL 33.000708). As far the authors know there are no commercial businesses that commercially exploit this technology. The main disadvantage of these devices in relation to the present invention is that they are developed on planar technology, which requires expensive coupling, encapsulation and packaging systems to obtain the information. Besides, they require well-developed micromanufacturing techniques since resonant cavities are highly sensitive to manufacturing defects, fewer devices can be integrated than with the alternative technology proposed in the present invention and the capacity for creating high measuring cadence devices is more complex.

### 4.- Refractometric biosensors based on ohotonic crystals:

It can be noted the *Photonic Crystal Microcavities (PCW)*. These biosensors use the properties of photonic crystals wherein there exist a large number of patents (for example, US2008252890, US2008219615) and applications for creating interferometric devices or high quality factor resonant cavities by including defects in the photonic structure (for example, Ultracompact biochemical sensor built with two dimensional photonic crystal microcavity, Opt. Lett. 29, (10), pp. 1093-1095, 2004. doi:10. 1364/0L.29.001093*).* High sensitive values are reported, though the necessary manufacturing requirements for their commercial application seem to be extremely demanding, apart from the negative effect in other aspects such as integration, encapsulation, packaging to achieve cost reduction and high measuring cadence.

Finally, it should be highlighted that the research activity in the field of label-free detection as a method for molecular recognition is very intense since avoiding labelling through fluorescence, radioactivity or magnetism will drastically improve the cost per analysis, the detection quality and the capacity for developing new medicines and the identification of diseases with the impact that this will have in the future. Innovative photonic structures are being continuously proposed such as, for example, high quality factor resonant cavities even for the detection of a single biomolecule (Label-Free, Single-Molecule Detection with Optical Microcavities, Science Volume 317, page 783, 2007*)*.

The present invention is novel and improves the state of the science and technology in the current market in the field of integrated optical biosensors in relation to the last advances due to its approach towards technology Integration to achieve that the label-free biodetection could be exploited at both the disposable biochip level and the high measuring cadence detection level in large substrates, to offer some sensitivity and robustness metrics that no current system is able to provide, due to the fact of combining advanced optical techniques of vertical interrogation and novel biophotonic structures as it will be explained below.

Since the measuring system is based not only on the design of high sensitivity biophotonic structures, but also on advanced optical interrogation techniques, it is convenient to note that, at present, there exist commercial firms that partially offer complementary optical interrogation techniques used either in the field of research or in the manufacturing of devices in the micro-nano electronics sector, but none of them is an integrated system for the analysis of biochips and photonic structures for labelling-free bioassays. Among them, we can highlight spectrometry, that can be made at the level of microdomains, by means of either dispersive spectrometers where there exist several manufacturers offering this technique (for example, *HORIBA Jobin Yvon* www.jobinyvon.com), or by Fourier Transform where there also exist suppliers that offer high quality equipment (for example, *Bruker-Optics,* www.brukeroptics.com), or even micro spectrometers, though these usually offer very low resolution to be used for biodetection. We can also highlight the number of manufacturers that offer optical systems based on confocal technology such as, for example, Olympus, Leica, Zeiss, Nikon, Perkin-Elmer, Nanofocus AG, etc. that allows observing micro and sub-micro domains of samples for their analysis. As regards the existence of equipment that integrates more than one optical technology for high cadence applications, we can highlight the suppliers in the sector of microelectronics, for example, *KLA-Tencor* (http://www.kla-tencor.com/) that commercialize a wide variety of systems for the online optical characterization in the manufacturing of devices in the sector of microelectronics and semiconductors. Optical technologies such as ellipsometry, spectrometry and reflectometry on the basis of the angle of incidence are very useful and have been adapted to achieve high cadence and to assess hundreds of devices and structures within micro domains in wafers usually made of silicon, being it possible to assess large substrates such as, for example, 300 mm diameter silicon wafers. Next, reference is made to some of these patents such as: *"Method and apparatus for evaluating the thickness of thin films (*EP0549166*"), "High revolution ellipsometric apparatus (*EP0396409*)"* and *"Self-calibrating beam profile ellipsometer (*US2004233436*)".* The existing patents in this field only refer to the sector of microelectronics, only to characterize physical properties of electronic structures by reflection usually manufactured with dielectric materials and in general Si technology, and on Si wafer substrates (they do not contemplate transmission as in the present invention), and it has never been contemplated, nor has it been designed, advanced measuring techniques of vertical assessment for bio-detection based on the analysis of responses of bio-chips formed by interferometric and biophotonic resonant structures.

In conclusion, as far as the authors have found there does not exist any bio-detection system based on the concept described in the present invention, neither has it been reported, and at present no manufacturer offers an integrated optical detection system for the analysis of micro and sub-micro domains that integrate transmission and reflection solutions for analysis by means of ellipsometry, spectrometry and angle resolved transmittance/reflectance, and let alone for the analysis of resonant and/or biophotonic Interferometric cells as an optical detection system for labelling-free bioassays. Besides, in the present invention it is proposed the use of a CCD-type matrix detection system that will allow for spatially analyzing all the reflection and transmission profile to obtain more precise information and with more resolution of the phase difference of the polarized waves, spectral response and angle resolved reflectance/transmittance. The optical analysis of the reflection and transmission profile of the biophotonic micro cells, never before contemplated, will mean a significant advance in the development and efficiency of label-free biodetection. Said optical biodetection system is neither devised nor developed by any manufacturer at present.

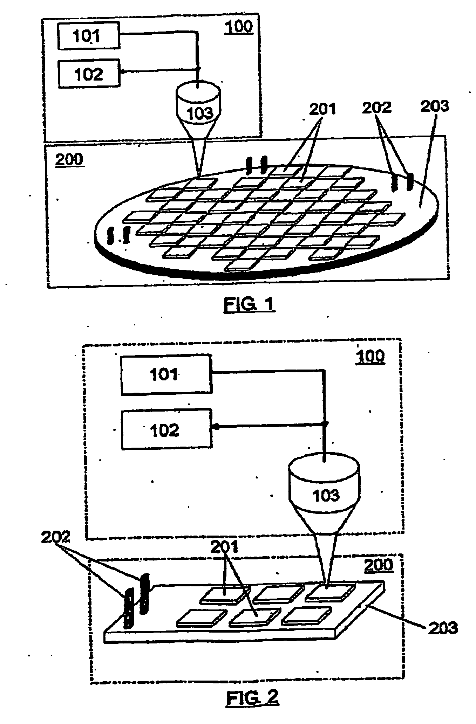

The article "Resonant Cavity Imaging: A Means Toward High-Throughput Label-Free Protein Detection; Bergstein D A; IEEE JOURNAL OF SELECTED TOPICS IN QUANTUM ELECTRONICS, VOL. 14, NR. 1, pages 131 - 139; 20080101" discloses (Figs. 1, 4, 7) a resonant cavity imaging biosensor (RCIB) using an optical technique for detecting molecular binding interactions label free at many locations in parallel that employs an optical resonant cavity for high sensitivity. According to this technique near-infrared light passes resonantly through a Fabry-Perot cavity comprised of upper and lower dielectric reflectors made of alternate Si/SiO2 layers and the wavelength is swept whereby a wavelength shift in the local resonant response of the optical cavity indicates binding. The transmitted intensity at thousands (e.g., 16384) of pixel locations can thus be recorded simultaneously.

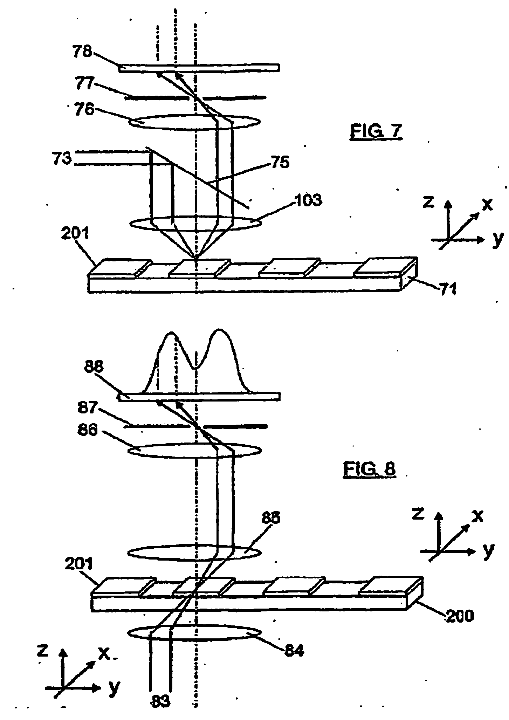

Fig. 1 discloses how independent simultaneous measurements can be performed using a device comprising two partially reflecting surfaces positioned facing one another to form a Fabry-Perot cavity. The capturing agents are patterned on one of the reflecting surfaces. A collimated beam of light is incident on the cavity through one of the reflectors, and the wavelength is swept in time. A camera images the transmitted intensity mapping the resonance characteristic from each location along the planar cavity surface to a pixel on the camera. Target molecules bound to their corresponding capturing agents fixed to the reflector surface shift the local resonant condition of the cavity. The number of spots evaluated simultaneously is essentially limited by the number of pixels available on the camera, and thus, can scale readily to achieve very high throughput. The resonant cavity reflectors consist of alternating layers of Si and SiO2. Fig. 7 shows binding reactions imaged in three flow channels (#1,#2,#3) indicating different signal strengths when viewed facing the substrate surface.

### DESCRIPTION OF THE INVENTION

The present invention consists of a novel optical detection system for high-sensitivity labelling-free bioassays as defined according to the appended claims. Said system is based on optical vertical interrogation biophotonic cells manufactured with micro-nanomanufacturing technology that may be integrated into large substrates (for example, silicon, glass wafers, etc.) for high measuring cadence, or individually for the production of disposable chips. Said cells offer a high-sensitivity response to tiny changes of the refractive index of the biomolecules attached to the cavities of their structures. The interrogation is performed by vertically and simultaneously analyzing the reflection and/or transmission profile of the light beam of the biophotonic cells by means of ellipsometry, spectrometry, and transmittance or reflectance techniques according to the incidence angle, avoiding the high cost that the encapsulation of the conventional photonic chips involves by avoiding the fiber optic-waveguide coupling.

More specifically, the present invention describes the development of a high-sensitivity optical biosensing system that avoids the need for labelling (for example, fluorescence, radioactivity or magnetism) and also offers high measuring cadence for molecular recognition, such as for example, the detection of pathogens or proteins by means of antibody-antigen affinity reactions. The key benefits and features of the present invention are based on a new concept of biosensing system that combines, on the one hand, the use of advanced optical characterization techniques that allow for vertical interrogation in micro and sub-micrometric domains and, on the other hand, the development of highly-sensitive interferometric and/or biophotonic resonant structures for increasing the capacity for detecting tiny variations of the refractive index. Besides, materials that may be used for making the sensitive biophotonic cells are compatible with large-scale micro-nano manufacturing processes such as silicon dioxide, silicon nitride, polydimethylsiloxane (PDMS), SU8 resin, titanium dioxide, or any other dielectric, as well as thin films made of metal such as aluminum, copper, etc.

By simultaneously observing the reflection or transmission profile of several complementary optical interrogation techniques (for example, ellipsometry, spectrometry and angle resolved reflectance or transmittance) and the application for labelling-free bioassays of biophotonic structures it will be possible to determine with higher reliability and sensitivity the molecular interaction with the immobilized molecular receptors in the sensitive areas of the biophotonic cells. The fact that the interrogation is vertically and simultaneously made by means of several high-sensitivity optical detection techniques, avoids measuring uncertainties and ambiguities usually present in the optical detection systems.

The biosensing system proposed works as a system that is ultra-sensitive to tiny variations of the refractive index during the molecular recognition process, and thus has dear potential applications in those fields in which a bioassay may be developed, such as, for example, the biomedical, biochemical, pharmaceutical, clinical, environmental and security sectors, since the variations of the refractive index of the molecular interactions are within the range of refractive indexes that may be easily detected with this system.

The biosensing optical system proposed also solves a problem inherent to the conventional photonic chips micro-nano manufactured in planar technology, by offering a low-cost solution for integrating, encapsulating and packaging the chips. This is so because the interrogation of biophotonic sensitive cell is vertical, either in reflection or transmission, avoiding the need for the use of expensive fiber optic-waveguide coupling systems (for example, inverted narrowing guides and couplers based on diffraction networks) to obtain information. To this end one or several excitation sources will be used within the visible and infrared range, coherent or not, such as for example, a beam of laser light.

Thus, the interrogation of the bio-sensitive cells will be made by means of a highly focused light through a high numerical aperture optical objective, allowing for the analysis in micro and sub-micro domains. The size of the domain of analysis is determined by the diffraction limit, which means that for longer wavelengths domains will be larger than for shorter wavelengths (for example, for a 633 mm wavelength and a standard microscope objective having a 0.8 numerical aperture it will be possible to analyze domains of about 0.8 micrometers). It must be noted that the high numerical aperture objectives will allow for directly observing the reflection or transmission profile through a CCD-type matrix detector, which will also mean simultaneously obtaining the angle resolved transmittance or reflectance. In turn, if the excitation light is polarized, it will be possible to obtain from the same profile information regarding the polarization state of the reflected and transmitted light, thus simultaneously obtaining ellipsometric information to the angle resolved transmittance and reflectance. Finally, in order to obtain the spectral response it will be necessary to have a multi-wavelength light source and a, for example, grating-type dispersive element for the detection of the response of the resolved reflectance/transmittance in wavelength.

The domains where the bio-sensitive cells are integrated will be directly manufactured on disposable chips, or on large substrates such as, for example, wafers made of silicon, glass, or any other material. In turn, the system will be able to have an image and geometry recognition sub-system that will allow for placing the measuring head in the bio-sensitive areas with enough resolution and precision, making it possible not only to increase the number of cells per surface unit, that is to increase the bio-sensitive cell integration, but also to make the cadence and access to the biophotonic sensitive cells to be automatic. It should be noted that in order to increase the system robustness, it would also be possible to use the optical response both in reflection and/or transmission of the biophotonic cells as a control information and tool for determining the correct placement since the symmetry profile of the optical intensity both in transmission and reflection may be designed from the geometry of biophotonic structures. For example, in the case of biophotonic cells formed by periodic pattern micropillar network, the reflection/transmission profile will also be symmetric when the optical head is well positioned.

Ultimately, by making this system, it will be possible to achieve that the routine of measuring and analyzing analytes be more efficient from the point of view of costs and adapted to make hundreds of measures on a single or several analytes. Therefore, the system offers the unique capacity for multiple measures of either a single or several analytes. Besides, the simultaneous use of several optical interrogation techniques in a few seconds makes the system more robust allowing the system to be capable of offering a high measuring cadence and productivity compared to other alternative analysis techniques for bio-detection.

The system will be capable of measuring both disposable bio-chips for applications where the demand of analysis requires proximity to the point of care (for example, a health center), or to the point of interest in general (for example, analysis of pollutants in a river), and large substrates for high measuring cadence applications for the development of, for example, new medicine and pharmaceutical receptors for the development of therapeutic targets.

The optical detection system for labelling-free bioassays proposed in the present invention offers an unprecedented detection system being a holistic solution that includes all the features offered, on the one hand, by the biophotonic cells and, on the other, by the proposed advanced optical interrogation techniques:
- *Biophotonic cells:* The photonic structures designed that form the biophotonic cells of vertical optical interrogation will offer the advantages of the above mentioned physical phenomena such as resonance, interferometry. Besides, they may offer the capacity of confining and concentrating the electric field in those sensitive zones where biomolecules will be immobilized to improve sensitivity. In the present invention, the biophotonic cells formed by a periodical pattern of resonant micropillars will concentrate the evanescent electric field on the surface of said pillars where biomolecules will be immobilized, thereby making the response of the resonant micropillars to noticeably increase the sensitivity of the biophotonic cells.
- *Vertical interrogation optical techniques:* The efficiency of the high-resolution optical techniques, capacity of characterization and detection such as the ones proposed for the vertical interrogation of the biophotonic cells will offer a unique and advanced solution because the high-sensitivity response of the biophotonic cells is combined with the optical analysis techniques that are more sensitive to variations of the refractive index. The resonant and/or interferometric optical response to the reactions of affinity and molecular recognition of the corresponding biophotonic cells, will be amplified when they are interrogated using the change of the light polarization (allipsometry). the response of reflectance and/or transmittance resolved in: wavelength (spectrometry) and in angle of incidence. Besides, the proposed techniques will allow: the analysis in micro and sub-micro domains, the localization of said domains by means of image and geometry recognition, and thus, the capacity for integrating multiple sensitive cells per area unit. Linked to these advantages are the time of analysis in a few seconds and the robustness of simultaneously analyzing each biophotonic cell with high-sensitivity optical techniques, making the optical interrogation process more reliable.

Ultimately, the unique concept proposed in the present invention will allow developing label-free bio-detection systems that are competitive in terms of industrial commercialization, sensitivity, measuring cadence, robustness and cost.

In the present invention, the vertical interrogation cavities that are proposed offer the following advantages:
*1- High sensitivity,* since they use the interferometric and resonant phenomena of the photonic structures proposed when they are assessed by high-sensitivity measuring techniques (for example, ellipsometry).
*2- Robustness,* since the photonic structures are simultaneously analyzed with several measuring techniques, which reduces measuring uncertainty and ambiguity in the interrogation process.
*3- Simple coupling system,* the vertical assessment in micro and sub-micro domains is easily made using a high numerical aperture microscope objective, which allows a simple access to the structures, where a simple optical system for image recognition may be implemented for the localization of the measuring areas.
*4- Cost reduction,* since it harnesses all the advantages of the micronano manufacturing technology and allows the integration of multiple devices for the simultaneous analysis of several analytes. Besides, the cost of analysis is reduced since the detection cells may be highly integrated, for example, it is viable to integrate 100 sensitive cells per 1 cm², which would mean a 10 eurocent cost per analysis, that is negligible compared to the reactive and biological material cost. Besides, not having to use labelling techniques (for example, fluorescence or radioactivity) in the preparation of the samples also reduces the cost Finally, this optical detection system in micro and sub-micro domains allows for analyzing extremely small volumes of analytes (far below the nanotitres) in comparison with the current systems (in the microlitre range) also reducing the amount of reactives used to carry out the analysis.
*5- High measuring cadence,* the duration of an optical measure is a short interval of time, in a few seconds, and the development of the current technology allows that the movement for placing the optical probe may go device by device for its assessment in a very precise way and within a short time.
*6*- *Applications:* it has multiple applications from the bio-detection point of view, since they depend on the corresponding biological applications that are constantly being developed. Therefore, the detection system may be applied to all those applications in which a bioassay may be developed, and thus as any bio-detection system it will be relevant in high impact sectors such as the clinical, biomedical, environmental, pharmaceutical, security, etc. sectors. It is worth highlighting that the system of the present invention will be able to measure both in large substrates for applications where the measuring cadence is essential for increasing efficiency, reducing the cost and the time in the development of, for example, new medicines, as well as for applications in which the point of analysis requires a complicated access (for example, for environmental applications) or for reducing the time and cost when it is possible to make the analysis in the proximity of the point of care such as, for example, a health center. This mitigation may be made by means of low cost disposable bio-chips.

### BRIEF DESCRIPTION OF THE DRAWINGS

The following is a brief description of a series of drawings which will help understand the invention better relating to an embodiment of said invention which is presented as a non-limiting example thereof.
- FIGURE 1. Schematic representation of the label-free optical bio-detection system at large substrate scale such as silicon or transparent glass wafers.
- FIGURE 2. Schematic representation of the optical bio-detection system at scale of disposable chip.
- FIGURE 3. View of an example of a bio-sensitive photonic cell based on a *Fabry-Perot* type resonant cavity of vertical interrogation by reflection.
- FIGURE 4. View of an example of a bio-sensitive photonic cell based on a *Fabry-Perot* type resonant cavity of vertical interrogation by transmission.
- FIGURE 5. View of an example of a bio-sensitive photonic cell based on resonant micropillars of vertical interrogation by reflection according to the invention.
- FIGURE 6. Example of a bio-sensitive photonic cell based on a network of resonant micropillars of vertical interrogation by transmission according to the invention.
- FIGURE 7. Example of an optical system of interrogation of biophotonic cells based on the analysis of the reflection of incident light.
- FIGURE 8. Example of an optical system of interrogation of biophotonic cells based on the analysis of transmission.
- FIGURE 9. Bio-detection by means of the angle resolved reflectance response for a Fabry-Perot type resonant cavity biophotonic cell. The resonator response is represented for two situations: with no biomolecular anchoring (photonic response of the cell) and for a certain molecular recognition equivalent to 0.01 Refractive Index Units (R.I.U.).
- FIGURE 10. Bio-detection by means of the spectrometry response for a Fabry-Perot type resonant cavity biophotonic cell. The movement of the resonance peak on the basis of wavelength is represented for two situations: with no biomolecular anchoring (photonic response of the cell) and for a certain molecular recognition equivalent to 0.01 R.I.U. (Refractive Index Units).
- FIGURE 11. Bio-detection by means of the ellipsometric response for a Fabry-Perot type resonant cavity biophotonic cell. The difference of phase between the vertical and the horizontal polarization states, on the basis of the molecular recognition generated by the increase in the refractive index, is represented.
- FIGURE 12. Example of a micro manufacturing Process of a vertical assessment biophotonic cell.
- FIGURE 13. Schematic representation of an example of complete integration of a vertical optical interrogation of a biophotonic cell.

### PREFERRED EMBODIMENT OF THE INVENTION

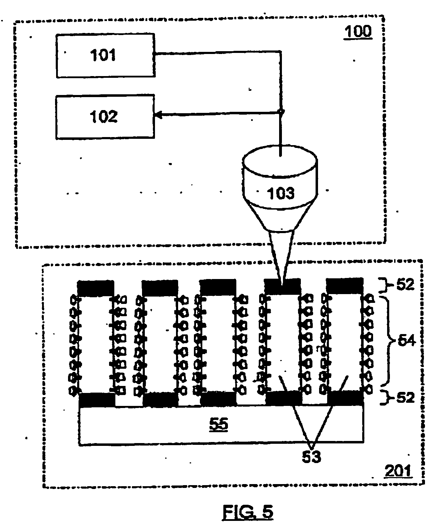

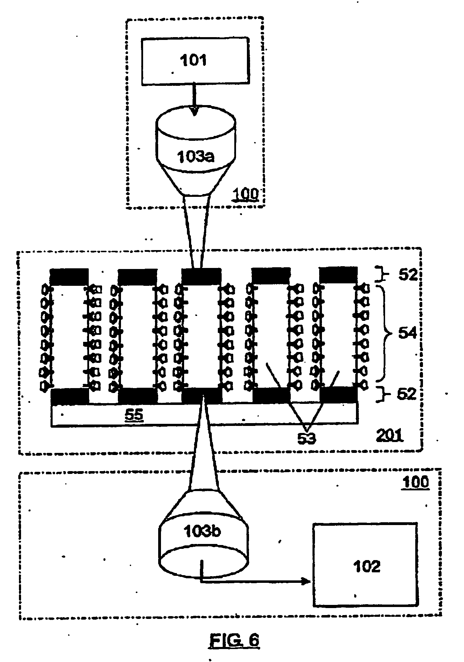

As it has been indicated above, it is the object of the present invention the development of a high sensitivity and capacity optical bio-detection system for labelling-free bioassays. The key benefits and features of the present invention are based on a new concept of biosensing system that combines on the one hand the use of advanced optical characterization techniques that allow the vertical interrogation in micro and sub-micrometric domains and on the other hand the development of biophotonic cells (as it is observed in Figures 5 and 6) that will be extremely sensitive to tiny variations of the refractive index that may be produced by the anchoring of biomolecules on the sensitive surfaces of the cavities of said biophotonic cells, making possible the molecular recognition and detecting, for example, antibody-antigen affinity reactions in a considerably sensitive way- The biophotonic cells may be manufactured with materials that are compatible with the large-scale micro-nano manufacturing processes such as silicon oxide, silicon nitride, polydimethylsiloxane (PDMS). SU8, or any dielectric, as well as thin films made of metal such as aluminum, gold, etc.

By the simultaneous spatial observation of the intensity of the reflected (Figure 7) or transmitted light (Figure 8) of for example, three complementary techniques of optical interrogation (ellipsometry, spectrometry and angle resolved reflectance or transmittance) and the amplification for labelling-free bioassays of the resonant or interferometric biophotonic vertical interrogation structures integrated in optofluidic cells, it will be possible to determine with higher reliability and sensitivity the molecular interaction with the immobilized molecular receptors in the sensitive areas, avoiding all the uncertainties and ambiguities of analysis usually present in the optical detection systems.

The biosensing optical system proposed works as a system that is ultra-sensitive to tiny variations of the refractive index generated by the biomolecular interaction of the corresponding assay, and thus has clear potential applications in those fields in which a bioassay may be developed, such as, for example, the biomedical, biochemical, pharmaceutical, clinical, environmental and security sectors, since the variations of the refractive index of the molecular interactions are within the range of refractive indexes that can be easily detected with this system.

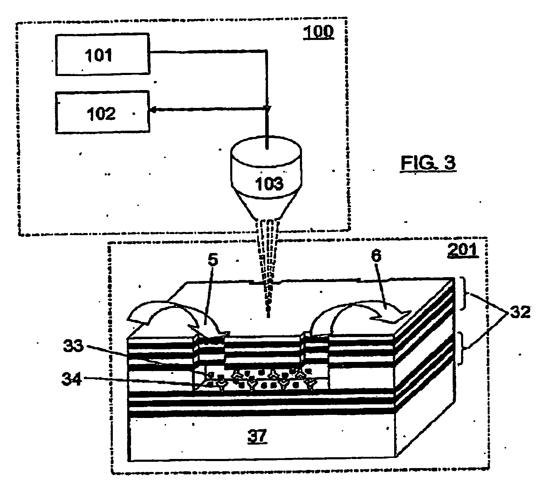

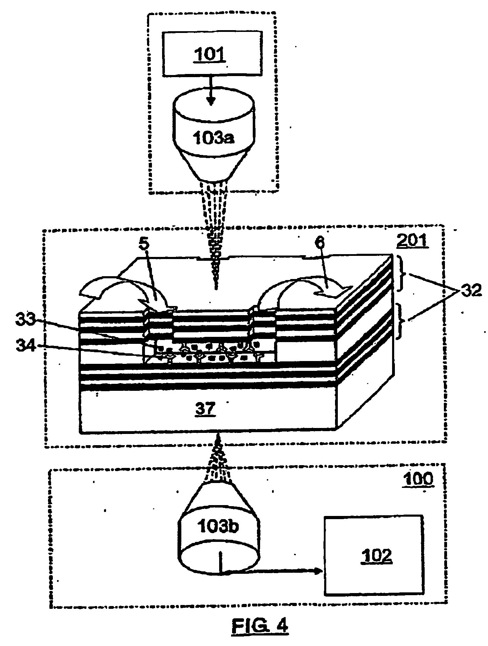

The optical biodetection system proposed also solves a problem inherent to the conventional photonic chips manufactured in planar technology by offering a more efficient and low cost solution for the integration, encapsulation and packaging of the bio-chips as the interrogation of the biophotonic sensitive cell is vertical, either in transmission (Figures 4 and 6) or in reflection (Figures 3 and 5) avoiding the expensive fiber optic-waveguide coupling systems necessary in the photonic bio-chips implemented in planar technology (for example, inverted narrowing guides and couplers based on diffraction networks) to obtain the information. To that end (Figures 7 and 8) a beam of laser light or another source of light, coherent or not, highly focused by means of a high numerical aperture objective will be used, allowing the analysis in micro and sub-micro domains both in disposable chips (Figure 2) and in large substrates (Figure 1) such as silicon wafers. The system may have an image recognition system included in the detection system to locate and place the measuring head in the detection areas, thus making possible the automatic cadence and access to the biophotonic sensitive cells. The collected profiles of intensity of reflected (see 78 in Figure 7) and transmitted (see 88 in Figure 8) light may also be used as adjustment tools for determining the correct placement due to the spatial symmetry of the optical intensity profile (Figures 7 and 8). This is the case since the biophotonic cells are designed so that the optical response of transmission or reflection is symmetric, in view of the micropillar periodic networks (Figures 5 and 6), of the biophotonic cell. These techniques will allow the density of sensitive cells to be increased to provide a bio-detection optical system capable of allowing the routine and sequence of analyte measuring to be adapted to make hundreds of measurements on a single or several analytes. The simultaneous use of several optical interrogation techniques in a few seconds will allow the system to be able to offer a high measuring cadence and productivity compared to other alternative analysis techniques, making the process more efficient.

The system will be capable of measuring both disposable bio-chips for applications in which it is necessary to bring the analysis closer to the point of demand, for example: the point of care (clinical application), the point of environmental detection (for example, detection of water purity, pesticides, etc.), and in large substrate for applications where the high measuring cadence may be crucial, such as, for example, the development of new drugs. Thus, more specifically, the optical detection system for high-sensitivity labelling-free hioassays object of the present patent of invention comprises
(i) an optical measuring system that comprises at least an excitation source (101), and optical head (103, 103a, 103b), and optical means for the detection (102) of a signal coming from the optical head (103, 103a, 103b),
(ii) an element for the interrogation of a fluid having multiple analytes comprising a plurality of biosensitive cells (201), wherein each of the biosensitive cells (201) is formed by a periodical pattern of resonant micropillars and each biosensitive cell comprises:
   ∘ a substrate (55),
   ∘ a plurality of resonant cavities (53), each cavity defined by one of said micropillars with its base resting on the substrate such that the space between micropillars receives the fluid,
   ∘ a plurality of Bragg reflectors, at least two per resonant cavity (53), located respectively at each end of the micropillar, and
   ∘ a plurality of molecular receptors (54) attached to the lateral surfaces of the micropillars so as to be in contact with the fluid,
   wherein said optical head is arranged to analyze each one of said biosensitive cells and the analytes contained therein.

The optical head (103) is arranged in such a manner as to allow the analysis of each one of said biosensitive cells (201) and the analytes contained therein.

In Figure 1 said element for the integration of multiple analytes (200) is a large element, while in Figure 2 said element (200) is a bio-chip (20) containing the same elements of element (200) in a reduced space.

In Figure 3 it is possible to observe an example of a blosensitive cell (201) based on, in this example, a *"Fabry-Perot"*-type resonant cavity of interrogation by obtaining the reflection profile from which the angle and wavelength resolved reflectance information is obtained, and where the ellipsometric information is obtained by analyzing the same reflection profile to obtain the difference of phase of the horizontal and vertical polarization states. The angle resolved reflectance and ellipsometric information is simultaneously obtained, while the spectrometric information is obtained on a continuous basis. Said biosensitive cell (201) comprises, at least:
(a) a plurality of Bragg reflectors (32);
(b) a resonant cavity (33);
(c) a plurality of biomolecular receptors (34), preferably proteins (for example, antibodies lantigen) immobilized in the cavity. The analyte enters through a fluid inlet (35) and exits through a fluid outlet (36); the molecular recognition is made and detected when the analyte is introduced and distributed in the cavity (33); being, besides, said elements (32, 33, 34) integrated on the substrate (37).

In Figure 4 on the same bio-sensitive cell (201) of Figure 3 it is observed how the interrogation is produced by transmission, where the angle and wavelength resolved transmittance information is obtained, and where the ellipsometric information is obtained by analyzing the same transmission profile to obtain the phase difference of the horizontal and vertical polarization states. The angle resolved transmittance and ellipsometric information is simultaneously obtained, while the spectrometric information is obtained on a continuous basis. In this arrangement, the optical beam emitted by an optical transmitter head (103a) connected to an excitation source (101) is received by an optical receptor head (103b).

Figure 5 shows an embodiment of the biosensitive cell (201) based on a periodic network of resonant micropillars and of a vertical interrogation by reflection, where the angle and wavelength resolved reflectance information is obtained, and where the ellipsometric information is obtained by analyzing the same reflection profile to obtain the phase difference of the horizontal and vertical polarization states. The angle resolved reflectance and ellipsometric information is simultaneously obtained, while the spectrometric information is obtained on a continuous basis. Said cell (201) comprises, at least:
(a) a plurality of resonant cavities (53);
(b) a plurality of molecular bio-receptors (for example, antibodies/antigen) (54) attached to the surface of the resonant cavities (53), the molecular recognition is produced and detected when the analyte is introduced and distributed in the periodic network of resonant micropiltars of the biophotonic cell (201);
(c) a plurality of Bragg reflectors (52), at least two per resonant cavity (53); being said elements (52, 53, 54) also deposited on the substrate (55).

In Figure 6 on the same biosensitive photonic cell (201) of Figure 5 it is observed how the interrogation is produced by transmission, where the angle and wavelength resolved transmittance information is obtained, and where the ellipsometric information is obtained by analyzing the same transmission profile to obtain the phase difference of the horizontal and vertical polarization states. The angle resolved transmittance and ellipsometric information is simultaneously obtained, while the spectrometric information is obtained on a continuous basis. In this arrangement, the optical beam emitted by an optical transmitter head (103a) connected to the excitation source (101) is received by an optical receptor head (103b).

Figure 7 shows an example for making an optical system of vertical interrogation by reflection of biophotonic cells, which is valid for both large substrates and bio-chips. The light beam (73) from the excitation system (101) is reflected by a beam divider (75) and is focused on the biophotonic cell (201) by means of an optical head (103) comprising a high numerical aperture objective (74) capable of focusing on micro and sub-micro metric domains. Due to the high numerical aperture objective (74), the reflected light may be analyzed for several incidence angles, the range of incidence angles is determined by the objective aperture, being it feasible to analyze a range of incidence angles of ± 64° with a numerical Aperture objective of 0.9 easily available in the market. The reflected light from the biophotonic cell is passed through a lens (76) that is focused on the micro-metric hole (77) that functions as a spatial filter. The different incidence angles are simultaneously collected through a CCD-type matrix detector (78) and that form the reflection profile of response of the biophotonic cell. If the incident light is linearly polarized at 45°, the two components corresponding to the vertical (s) and horizontal (p) polarization states can be obtained. This may be achieved for example, by making the excitation light be linearly polarized and using a retardant and an analyzer for the detection. From this reflection profile it is possible to simultaneously obtain the angle resolved and the polarization state dependent reflectance, and hence the ellipsometric response.

Figure 8 shows a practical example for making an optical system of horizontal interrogation by transmission of biophotonic cells, which is valid for both large substrates and bio-chips. The light beam (83) from the excitation system (101) is focused on the biophotonic cell (201) that is housed in a transparent substrate (200) by means of an optical head (103) comprising a high numerical aperture objective (84, 85) capable of focusing on micro and sub-micro metric domains. Due to the high numerical aperture objective (84), the incident light may be analyzed for several incidence angles, the range of incidence angles is determined by the objective aperture, being it feasible to analyze for example, a range of incidence angles of ± 64° with a numerical aperture objective of 0.9 easily available in the market. The light transmitted from the biophotonic cell is passed through a lens (86) that is focused on the micro-metric hole (87) that functions as a spatial falter. The different transmitted angles are simultaneously collected through a CCD-type matrix detector (88) and that form the response transmission profile of the biophotonic cell. If the incident light is linearly polarized at 45°, the two components corresponding to the vertical (s) and horizontal (p) polarization states can be obtained. This will be achieved by means of a simple polarizer at the output of the light source. From this transmission profile it may be simultaneously obtained the angle resolved and the polarization state dependent reflectance by means of the use of, for example, a retardant and an analyzer for the detection, and hence the ellipsometric response.

As regards positioning, the biophotonic cells with Fabry-Perot biocavities (Figures 3 and 4) the positioning of the optical head will be less critical as the dimensions on the plane are larger. It is possible to make alignment marks so that an image recognition system may determine the measuring place, conventionally used in micro-manufacturing to locate particular structures. This is made by including particular motifs and patterns on the upper face of the chips. For the cells based on periodic networks of micropillars (Figures 5 and 6) the focusing dimensions are more critical, but this type of cells allows being helped to place the optical detection head by analyzing the symmetry of the intensity of the reflection profile.

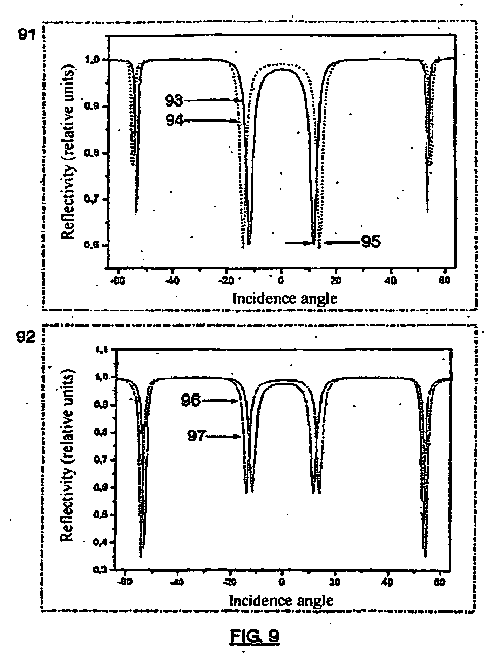

Figure 9 represents the theoretical detection response by means of the analysis of the reflection profile by angle resolved reflectance of a biophotonic cell (Figure 3). The biophotonic cell of Figure 3 is formed as a whole by Bragg reflectors and a cavity through which the different analytes will be introduced. Optically, said cell behaves as a Fabry-Perot-type resonator of biochemical detection. It may be observed that the angle resolved reflectance response where two reflection minima (95) appear due to the resonant response of the biophotonic cell, being the response very similar for the two polarization states: vertical (91) and horizontal (92). The angular position of said minima highly depends on the refractive index within the biocavity, this index will vary on the basis of the molecular interaction within the cavity. The curve (93, 96) represents the photonic response of the resonant cavity for vertical polarization, while the curve (94, 97) represents the response of the biophotonic cell for a percentage of recognized biomolecules equivalent to 0.01 Refractive Index Units (R.I.U).

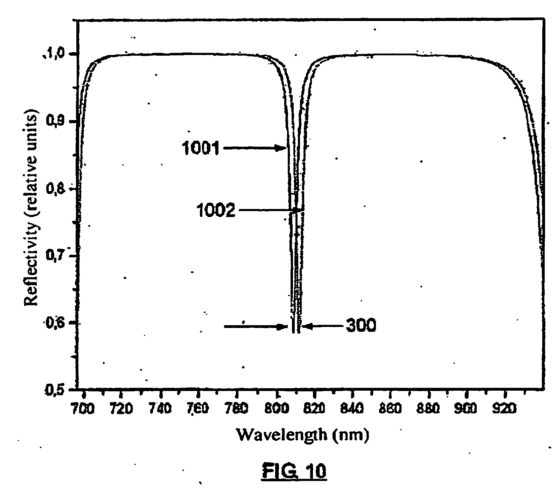

Figure 10 represents the theoretical detection response by means of the analysis of the reflection profile by wavelength resolved reflectance (spectrometry) for a biophotonic cell (Figure 3). For spectrometry there appears a highly steep resonance minimum (300) of about 810 nm, separated in more than 50nm from the following minimum. This minimum will move to larger or smaller wavelengths as the effective index of the biocavity varies due to the biomolecular interaction therein. The angular position of said minimum highly depends on the refractive index within the biocavity, and hence on the molecular recognition within the cavity. The curve 1001 represents the photonic response of the resonant cavity, while the curve 1002 represents the response of the biophotonic cell for a percentage of recognized biomolecules equivalent to 0.01 Refractive Index Units (R.I.U).

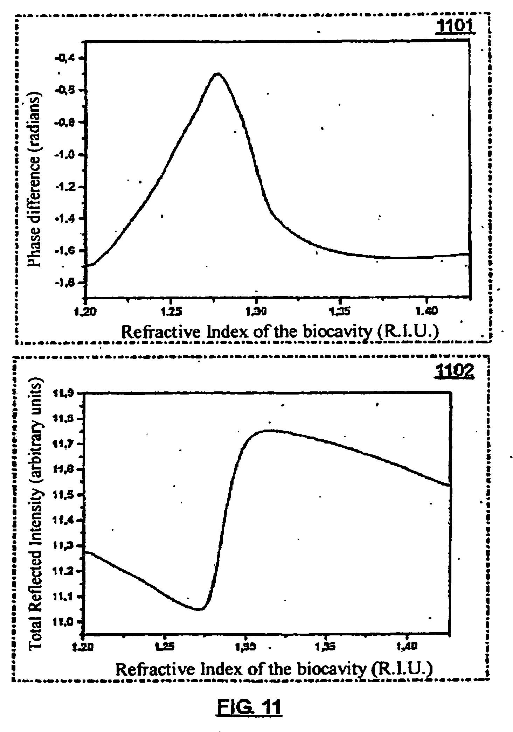

Figure 11 represents the theoretical detection response by means of the analysis of the reflection profile by ellipsometry. The figure shows the theoretical phase difference (1101) between the vertical (s) and horizontal (p) polarizations and the module (1102) for different refractive indexes of the cavity, which correspond to the concentration of recognized biomolecules. These curves are a theoretical estimate of the response and dynamic range of the biophotonic cell. At about n=1.33, said phase varies very quickly on the basis of the index of the biocavity.

For this cell, the calculated estimates show that for these structures the detection limit that may be achieved is 10⁻⁷ Refractive Index Units (R.I.U.). In the case of the biophotonic cell based on resonant micropillars (Figures 5 and 6), the theoretically obtained results are similar, though this type of structures allows concentrating the evanescent field on the surfaces where the biomolecules will be anchored. Besides, other biophotonic cells may be the object of the invention, such as for example, the sub-micro hole membranes that will allow the concentration of the evanescent field and a continual flow.

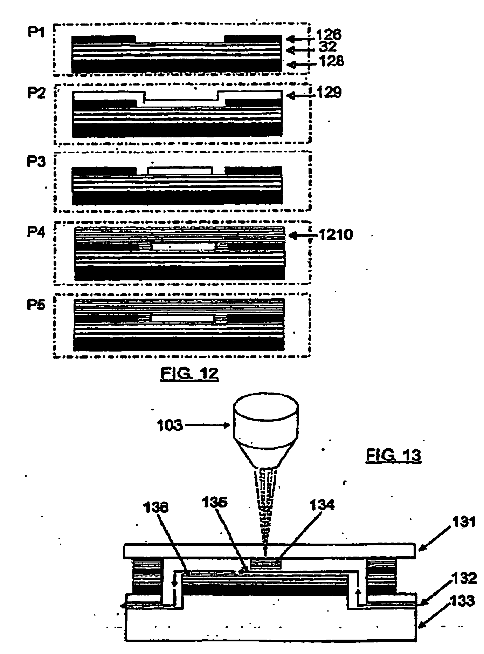

Figure 12 represents an example for manufacturing a biophotonic cell based on a Fabry-Perot-type resonant cavity (Figures 3 and 4) in five manufacturing steps (P1, P2, P3, P4. P5), though other biophotonic cells (Figures 5, 6) may be manufactured using similar micro-manufacturing methods. The following steps may be followed to manufacture this structure taken as an example (Figure 12):
- Manufacturing process P1: Manufacturing of the lower Bragg reflectors (32): The Bragg reflectors may be made in oxide pairs (SiO₂ of low refractive index) and silicon nitride (Si₃N₄ of high refractive index) on a substrate (128) to improve the response. The deposit of the reflectors has to ensure the uniformity of the deposited layers. For this process widely used techniques can be used such as LPCVD (Low-Pressure Chemical Vapor Deposition) or PECVD (Plasma-Enhanced Chemical Vapor Deposition) commonly used in micro-manufacturing technology. An intermediate amorphous silicon layer (126) is deposited after the manufacturing of the reflectors by means of PECVD, which will be used for the manufacturing of the biocavity using conventional photolithography techniques.
- Manufacturing process P2: Sacrificial layer (129). Once the cavity size is defined, there may be deposited a SiO₂ sacrificial layer in order to be subsequently eliminated and create a hollow cavity. PECVD may be also used.
- Manufacturing process P3: Definition of the cavity. Using the conventional photolithography techniques and the conventional etching techniques such as for example, plasma dry etching a thin SiO₂ plate may be defined only in the cavity. This SiO₂ layer will be subsequently eliminated to create a hollow cavity.
- Manufacturing process P4: Manufacturing of the upper Bragg reflectors (1210): The upper Bragg reflectors may be manufactured by means of alternative deposits of oxide and nitride by the same previously described techniques.
- Manufacturing process P5: Creation of the hollow cavity. For the elimination of the SiO2 sacrificial layer that defines the cavity there may be used wet etching that is conventionally used.

The fluid access of the different to the biophotonic cells may be simultaneously made using standard micro-manufacturing techniques and following similar processes. For example, in order to create the channels on the lower part of the wafer DRIE (Deep Reactive Ion Etching)-type deep dry etching techniques or wet etching may be used for example, using hydrofluoric acid (HF) or potassium hydroxide (KOH). Other possibilities include the use of transparent substrates, such as glass wafers, using and depositing the thin films made on polymer and resin such as for example, PDMS (Polydimethyl Siloxane) or SU-8 by means of precision spin coating techniques or using processing techniques suitable for the material used. The fluid channels at wafer level may be also processed by direct lithography laser ablation, for example, ablation processes by laser excimer having peak pulse and nano-seconds width, femtosecond lasers, by photolithography laser or by photo-polymerization, etc. Other micro-manufacturing techniques such as electron beam photolithography, etc. may also be used, though the structures of the biophotonic cells may be manufactured by mature micro-manufacturing techniques. Given the advance of micro-manufacturing technology during the past years, there exist several possibilities for making both the biophotonic cells and the channels and the fluid accesses in a competitive way.

Figure 13 shows a schematic technologically viable solution based on the standard micro-manufacturing techniques such as the ones that have been previously described. In this case a PDMS transparent layer (131) may be used, where there exists a fluid access channel (132) on a holder (133) in PMMA (Polymethyl Methacrylate) where the substrate rests (202) where the sensitive biophotonic cells are housed, in this case formed by a plurality of Bragg reflectors (32), upper (134) and lower (136) and a resonant fluid cavity (135) through which the corresponding analyte is introduced. The access to obtain the information of the response to the molecular recognition of the cavity is made by means of an optical head (103).

Finally, the biological detection application will be subject to the development of concrete bioassay for the detection of pathogens, particular proteins or DNA fragment for virus detection. One advantage of this biodetection system is that it may be used with most of the commercially existing bioassays or other to-be-developed ones, since they allow the continual flow and the possibility of sequentially introducing the necessary reactives to develop the corresponding biological application, hence it may also be used as a tool for the development of new bioassays. In order to carry out the biological application there has already been developed surface functionalization techniques where molecular bioreceptors may be immobilized and attached ((Label-JFee optical biosensing with slot-waveguides. Opt. Lett. 33, (7), pp. 708-71 0, 2008. doi:10. 1364/OL. 33.000708) which will be responsible for the biosensor selectivity. Once this process is made the biosensing system will be prepared for the final detection. The target analytes will be introduced through the corresponding fluid channel(s) and the molecular interaction or molecular recognition response will be optically monitored by the optical detection system. This response will be amplified due to the photonic response of the biosensing cells (Figures 9, 10 and 11). The simultaneous use of the optical techniques proposed (Figures 7 and 8) will drastically decrease the uncertainty and will make the system much more reliable. It will be possible to develop cognitive system oriented to users and professionals of the corresponding sector by means of software application which make the system very **easy to use.**

## Claims

1. An optical detection system for high sensitivity label free bioassays comprising the use of optical characterization techniques that allow resonant or interferometric vertical interrogation of micrometric and sub-micrometric domains of biophotonic cells comprising:
(i) an optical measuring system that comprises at least an excitation source (101), and optical head (103, 103a, 103b), and optical means for the detection (102) of a signal coming from the optical head (103, 103a, 103b),
(ii) an element for the interrogation of a fluid having multiple analytes comprising a plurality of biosensitive cells (201), wherein each of the biosensitive cells (201) is formed by a periodical pattern of resonant micropillars and each biosensitive cell comprises:
∘ a substrate (55),
∘ a plurality of resonant cavities (53), each cavity defined by one of said micropillars with its base resting on the substrate such that the space between micropillars receives the fluid,
∘ a plurality of Bragg reflectors, at least two per resonant cavity (53), located respectively at each end of the micropillar, and
∘ a plurality of molecular receptors (54) attached to the lateral surfaces of the micropillars so as to be in contact with the fluid,
wherein said optical head is arranged to analyze each one of said biosensitive cells and the analytes contained therein.

2. A system according to claim 1 wherein the element for the integration of multiple analytes comprises a plurality of fluid connections connected to the cells.

3. A system according to claim 1 **characterized in that** said excitation sources are laser-type sources or other sources coherent or not in visible and infrared wavelengths.

4. A system according to any of claims 1 to 3 **characterized in that** the element of integration of multiple analytes is a large substrate, of the silicon-, transparent glass- or the like wafer-type.

5. A system according to any of claims 1 to 4 **characterized in that** the element of integration of multiple analytes is a disposable biochip.

6. A system according to any claim 1 to 5 **characterized in that** the interrogation is by transmission, the optical head (103) is an optical transmitter head (103a) connected to the excitation source (101) whose beam is received by an optical receptor head (103b), connected to the optical detection means (102).

7. A system according to any claim 1 to 5 **characterized in that** the interrogation is by reflection, the optical (103) head is an optical transmitter-receptor head connected to the excitation source (101) whose beam is received by reflection by the optical head (103), connected to the optical detection means (102).

## Patentansprüche

1. Optisches Erkennungssystem für markierungsfreie Bioassays von hoher Empfindlichkeit, umfassend die Verwendung von optischen Charakterisierungstechniken, welche zur resonanten oder interferometrischen vertikalen Abfrage mikrometrischer und sub-mikrometrischer Domänen von Biophotonik-Zellen geeignet sind, umfassend:
(i) ein optisches Messsystem, welches mindestens eine Anregungsquelle (101) und einen Optikkopf (103, 103a, 103b) und optische Mittel zur Detektion (102) eines vom Optikkopf (103, 103a, 103b) kommenden Signals,
(ii) ein Element zur Abfrage einer Flüssigkeit mit mehreren Analyten, umfassend eine Vielzahl an biosensitiven Zellen (201), wobei jede der biosensitiven Zellen (201) durch ein periodisches Muster resonanter Mikrosäulen geformt ist und jede biosensitive Zelle umfasst:
∘ ein Substrat (55),
∘ eine Vielzahl resonanter Kavitäten (53), jede Kavität definiert durch eine der genannten Mikrosäulen, deren Basis derart auf dem Substrat gelagert ist, dass der Raum zwischen den Mikrosäulen die Flüssigkeit aufnimmt,
∘ eine Vielzahl Bragg-Reflektoren, mindestens zwei pro resonanter Kavität (53), jeweils an jedem Ende der Mikrosäulen angebracht, und
∘ eine Vielzahl von derart an den seitlichen Oberflächen der Mikrosäulen befestigten Molekularrezeptoren (54), um mit der Flüssigkeit in Kontakt zu sein,
wobei der Optikkopf angeordnet ist, um jede der biosensitiven Zellen und der darin enthaltenen Analyten zu analysieren.

2. System nach Anspruch 1, wobei das Element für die Integration mehrerer Analyten eine Vielzahl an mit den Zellen verbundenen Flüssigkeitsverbindungen umfasst.

3. System nach Anspruch 1, **dadurch gekennzeichnet, dass** die Anregungsquellen Laser-artige Quellen oder andere kohärente oder inkohärente Quellen mit sichtbaren-und Infrarotwellenlängen sind.

4. System nach einem der Ansprüche 1 bis 3, **dadurch gekennzeichnet, dass** das Element zur Integration mehrerer Analyten ein großes Substrat vom Typ Silikon, transparentes Glass oder ähnlichem Wafer-artig ist.

5. System nach einem der Ansprüche 1 bis 4, **dadurch gekennzeichnet dass** das Element zur Integration mehrerer Analyten ein Einweg-Biochip ist.

6. System nach einem der Ansprüche 1 bis 5, **dadurch gekennzeichnet, dass** die Abfrage durch Transmission erfolgt, der Optikkopf (103) ein optischer Transmitterkopf (103a) ist, der mit der Anregungsquelle (101) verbunden ist, deren Strahl von einem optischen Empfängerkopf (103b) empfangen wird, verbunden mit dem optischen Detektionsmittel (102).

7. System nach einem der Ansprüche 1 bis 5, **dadurch gekennzeichnet, dass** die Abfrage durch Reflektion erfolgt, der Optikkopf (103) ein mit der Anregungsquelle (101) verbundener optischer Transmitter-Empfängerkopf ist, deren Strahl durch Reflektion von dem mit dem optischen Detektionsmittel (102) verbundenen Optikkopf (103) empfangen wird.

## Revendications

1. Système de détection optique pour des essais biologiques libres d'étiquetage à haute sensibilité comprenant l'utilisation de techniques de caractérisation optique qui permettent l'interrogation verticale de résonance ou interférométrique de domaines micrométriques et sous-micrométriques de cellules bio photoniques comprenant :
(i) un système de mesure optique qui comprend au moins une source d'excitation (101), une tête optique (103, 103a, 103b), et des moyens optiques pour la détection (102) d'un signal provenant de la tête optique (103, 103a, 103b),
(ii) un élément pour l'interrogation d'un fluide ayant de multiples substances à analyser comprenant une pluralité de cellules bio sensibles (201), dans lequel chacune des cellules bio sensibles (201) est formée par un modèle périodique de micro-piliers à résonance et chaque cellule sensible comprend :
∘ un substrat (55),
∘ une pluralité de cavités résonnantes (53) chaque cavité définie par l'un desdits micro-piliers avec sa base restant sur le substrat de sorte que l'espace entre les micro-piliers reçoit le fluide,
∘ une pluralité de réflecteurs de Bragg, au moins deux par cavité résonnante (53), localisés respectivement au niveau de chaque extrémité du micro-pilier, et
∘ une pluralité de récepteurs moléculaires (54) fixés aux surfaces latérales des micro-piliers de manière à être en contact avec le fluide,
dans lequel ladite tête optique est disposée pour analyser chacune desdites cellules bio sensibles et les substances à analyser contenues dans celle-ci.

2. Système selon la revendication 1, dans lequel l'élément pour l'intégration de multiples substances à analyser comprend une pluralité de connexions de fluide reliées aux cellules.

3. Système selon la revendication 1, **caractérisé en ce que** lesdites sources d'excitation sont des sources de type laser ou d'autres sources cohérentes ou non dans des longueurs d'onde visibles et infrarouge.

4. Système selon l'une quelconque des revendications 1 à 3, **caractérisé en ce que** l'élément d'intégration de multiples substances à analyser est un grand substrat, du type tranche en silicium, en verre transparent ou analogue.

5. Système selon l'une quelconque des revendications 1 à 4, **caractérisé en ce que** l'élément d'intégration de multiples substances à analyser est une bio puce jetable.

6. Système selon l'une quelconque des revendications 1 à 5, **caractérisé en ce que** l'interrogation est effectuée par transmission, la tête optique (103) est une tête d'émetteur optique (103 a) reliée à la source d'excitation (101) dont le faisceau est reçu par une tête de récepteur optique (103b), reliée aux moyens de détection optique (102).

7. Système selon l'une quelconque des revendications 1 à 5, **caractérisé en ce que** l'interrogation est effectuée par réflexion, la tête optique (103) est une tête de récepteur - émetteur optique reliée à la source d'excitation (101) dont le faisceau est reçu par réflexion par la tête optique (103), reliée aux moyens de détection optique (102).
